# EUROPEAN PATENT APPLICATION

(11) **EP 4 708 317 A1**
(43) Date of publication of application: **11.03.2026**
(21) Application number: 24199428.4
(22) Date of filing: 10.09.2024
(51) Int. Cl.: G16H 40/20

(54) **DIAGNOSTICS PORTAL**

(71) Applicant: BIOTRONIK SE & Co. KG, 12359 Berlin (DE)
(72) Inventor: DÖRR, Thomas, 12437 Berlin (DE)
(74) Representative: Biotronik Corporate Services SE

(57) **Abstract**

A method for central medical service mediation, the method comprising: providing, by a user device, a medical service request to a medical platform; providing a user legitimation key to the medical platform; wherein the user legitimation key is associated with the medical service request and/or the user device; and wherein the user legitimation key is configured to allow for a secure transmission of the medical service request by the medical platform; and receiving, by the user device, a response based at least partly on the medical service request.

## Description

The present invention relates to methods, computer programs and systems for central medical service mediation.

Currently, medical service workflows are highly inefficient and time-consuming. For example, a patient may require medical services from multiple clinicians. The patient may typically visit these multiple clinicians in multiple face-to-face visits (often with long breaks between subsequent visits, e.g., due to scheduling issues). In some examples, in a first face-to-face visit, a first clinician may perform a first medical service (e.g., a diagnostic service) wherein they might, e.g., acquire first medical patient data on the patient (e.g., an X-ray image). The resulting diagnosis may lead to the decision than a second medical service (e.g., a further diagnostic service based on the acquired first medical patient data) is required. Typically, in a second face-to-face visit, a second clinician may perform said second medical service. Based thereon, it may be decided that a third medial service (e.g., a therapeutic service like a surgery) may be required. Typically, the patient may therefore attend a third face-to-face appointment with a third clinician, i.e., the clinician intended to perform said third and fourth medical service (e.g., in order to plan the fourth medical service) and then later attend the fourth face-to-face appointment (e.g., the surgery).

This exemplary workflow illustrates the time- and resource-consuming nature of current workflows. Due to the limited times at which both, the clinician and the patient may be available for a face-to-face visit, the whole process may extend over weeks or months even though the actual work might be done in a total time ranging from minutes to hours. Additionally, these require making multiple appointments (often by phone) which additionally slow down the process.

Therefore, there is still a need to further improve medical service workflows.

The aspects of the present invention meet the above need at least in part:
According to a first aspect of the present invention, a method for central medical service mediation comprises: providing, by a user device, a medical service request to a medical platform; providing a user legitimation key to the medical platform; wherein the user legitimation key is associated with the medical service request and/or the user device; and wherein the user legitimation key is configured to allow for a secure transmission of the medical service request by the medical platform; and receiving, by the user device, a response based at least partly on the medical service request.

Thereby, the workload associated with typical medical service workflows can be reduced significantly. In detail, the patient involvement and/or the need for face-to-face visits could be reduced. This may increase user, clinician, and patient satisfaction, save large amounts of time and thus resources. Further, the central mediation of medical services (e.g., independent from the locations and/or time zone of user, clinician and/or patient) may significantly accelerate said workflows. This may be particularly advantageous in cases where a health state of a patient may be critical such that any acceleration of said workflow may increase therapy success chances.

Importantly, the present invention achieves all of the above advantages without any drawbacks regarding patient data safety. Instead, only legitimized requests may be transmitted such that any subsequent step may automatically be legitimized. Providing such legitimation in addition to the associated medical service request was found to yield a particularly safe workflow. Additionally, it allows the implementation of already established measures (e.g., electronic health cards etc.) such that user, patient, and clinician acceptance and satisfaction may be increased, on the one hand, and legal patient data safety requirements may be met, on the other hand.

In one example, (a user, e.g., a member of the medical staff) may use the user device to provide the medical service request (e.g., an analysis of medical patient data by any qualified clinician). Once said medical service request has been accepted by a suitable clinician, the user may, via the user device, receive a response based at least partly on the medical service request (e.g., the response to the medical service request). The response may comprise one or more response portions, e.g., a first portion indicating that the medical service request has been accepted and a second portion providing the result of the performed medical service and/or information thereon.

Providing the user legitimation key may, e.g., be done by the user device and/or another patient or user device, e.g., a card-reader configured to read an electronic health card. Providing the user legitimation key may, e.g., comprise reading the electronic health card of a patient and/or the patient granting permission to the medical service request (e.g., by signing an electronic signature pad, by giving permission via a patient device (e.g., a mobile phone running a corresponding application and/or accessing a corresponding website)), and/or another suitable step. Thereby, it may be ensured that the patient legitimizes the medical service request which may ensure that the patient ultimately controls the distribution of, e.g., their medical patient data (in examples where the medical service request may comprise such medical patient data).

The user device, the clinician device, and/or the patient device may, e.g., comprise a smartphone, a tablet, a laptop, a desktop computer, and/or a smartwatch. Any data transmission described herein may, e.g., be realized via Wi-Fi, cellular networks, Ethernet, Bluetooth, satellite communication, similar options, and/or combinations thereof. For example, the present invention may utilize a communication protocol that allows for a certain degree of data safety for at least some of the data transmissions described herein. This protocol may comprise, e.g., HTTPS (HyperText Transfer Protocol Secure), SSL/TLS (Secure Sockets Layer/Transport Layer Security), VPN (Virtual Private Network), SSH (Secure Shell), and/or SFTP (Secure File Transfer Protocol).

A medical service may, e.g., comprise at least one of the following: a preventive care service (e.g., medical services aimed at preventing illnesses, such as vaccinations and routine check-ups), a diagnostic service (e.g., procedures and tests used to diagnose medical conditions, such as blood tests and imaging), a therapeutic service (e.g., treatments and interventions to manage or cure illnesses, such as physical therapy and prescription medications), an emergency service (e.g., immediate care for acute medical conditions or injuries, such as emergency room visits and urgent care), a rehabilitative service (e.g., services designed to help patients recover and regain function, such as occupational therapy and post-surgical rehabilitation).

In some examples, the user legitimation key may comprise a time window defining the time during which the medical service request can be provided to the medical platform by the user device to allow for transmission of the medical service request by the medical platform.

The definition of such time window was found to be a safe and efficient measure for the implementation of an efficient legitimation measure.

In some examples, the time window may, e.g., be associated with the moment in time when the user legitimation key is being provided. For example, the time window may, e.g., start at x1 minutes (wherein x1, may e.g., range from 180 min to 60 min) before providing the user legitimation key and/or end x2 minutes (wherein x2, may e.g., range from 180 min to 60 min) after providing the user legitimation key. Thus, the transmission of the medical service request may be allowed when it is provided in the time window [x1, x2], e.g., defined as described herein.

The time window may, e.g., be represented as times, e.g., in the format hh:mm:ss, dd.mm.yyyy (e.g., for the start and/or end time of the time window and/or for the duration of the time window from a certain start point), one or more representations of the start point, duration and/or end point of the time window in a non-temporal data format (e.g., a counter that may be associated with a clock and/or be recalculated into a time and/or any other similar or equivalent representation thereof.

The method may, for example, further comprise authenticating, by the user device, of a user. In some examples, the providing of the medical service request may be based at least partly on the authenticating.

In an exemplary embodiment, only an authenticated user may provide medical service request(s) and/or the authenticated user may be enabled to provide a medical service request selected from a first plurality of medical service request types and an unauthenticated user may be enabled to provide a medical service request selected from a second plurality of medical service request types. E.g., the first and second plurality of medical service request types may be different and/or the first plurality may comprise the second plurality and additional medical service request types (i.e., those that require authentication).

The authentication may, e.g., comprise logging into the medical platform by use of a user-specific identification and/or a user-specific password.

In some examples, the authenticating may comprise providing, by the user device, an authentication request to an authentication module of the medical platform; and being granted user access, via the user device, to at least a first part of the medical platform.

For example, the medical platform may provide different interfaces to different users, e.g., based at least partly on the authentication. For example, a user-interface may represent said first part when a user has successfully authenticated (e.g., by log-in). Said user-interface may, in this example, provide at least one filed for user input in order to provide a medical service request when wanted.

For example, when a user is, e.g., authenticated only as a patient (who might not be intended to provide the medical service request but merely providing the user legitimation key. The according interface shown to such patient (e.g., based at least partly on their authentication) may, e.g., comprise a section for providing said user legitimation key (e.g., by confirming a medical service request provided by another user). Additionally or alternatively, the medical service requests that may be provided by the patient may, e.g., be limited to requesting appointments with clinicians.

A second aspect of the present invention relates to a method for central medical service mediation. Said method comprises providing, by a clinician device, a clinician registration request to a medical platform; receiving, by the clinician device, one or more instructions for a clinician qualification check; and being granted clinician access, via the clinician device, to at least a second part of the medical platform upon successful execution of the one or more instructions for the clinician qualification check.

Thereby, a solution may provided that ensures that the medical platform can distinguish between clinicians of different qualifications and the qualification are indeed checked which may increase security of the whole workflow. These advantages may by particularly advantageous in synergy with the method according to the first aspect.

The successful execution may, e.g., comprise providing all required inputs (e.g., information and/or (confirming) documents) as per the instruction.

The clinician qualification check may, e.g., comprise providing documentation proving the qualification of the clinician associated with the registration request. The clinician qualification check may additionally or alternatively comprise a questionnaire comprising clinician-specific (e.g., name, location, qualification, offered medical services, offered medical service types, opening hours, availability status, and/or similar information) and/or medical questions for checking the qualification. Only those clinicians that pass said clinician qualification check may be granted access as described herein.

For example, a first clinician being a heart expert may provide a registration request as a heart expert. They may then receive an instruction for a first clinician qualification check (e.g., addressing heart-related aspects). For example, a second clinician being a radiologist may provide a registration request as a heart expert. They may then receive an instruction for a first clinician qualification check (e.g., addressing radiology-related aspects like which medical service they provide, e.g., X-ray, CT, MRI, etc.). The second part may respectively be different for these exemplary first and second clinicians.

In some examples, the second part of the medical platform may be based at least partly, on the clinician registration request, the instruction and/or the successful execution.

Thereby a higher degree of quality control and safety within the workflow is realized.

For example, the second part may be represented by a corresponding user interface shown to those clinicians that have access to the second part as described herein. For different clinicians, e.g., with different qualifications the respective interfaces may be different. Via said interface, the medical patient data associated with the medical service request may be provided in full or partly. E.g., the second part (as it may be related to the qualification of the clinician) may determine those features of the medical patient data that might be relevant for the respective clinician. When, e.g., the second part corresponds to the functionalities of the medical platform provided to a heart specialist, the medical platform may only provide those features of the medical patient data that might potentially be heart-related (i.e., the providing may be based at least partly on the second part). This may assist anonymization (e.g., the patient's address may be relevant for a home care service but not heart-disease related medical services and may be left out or not based on the second part of the medical platform/the clinician's qualification). This may advantageously free the user providing the request from selecting the exact features of the medical patient data that might be decisive for the clinician associated with the request. This degree of automatization may thus improve the reliability and user-friendliness of the medical platform.

The method may, e.g., further comprise receiving, by the clinician device, a medical service request from the medical platform (e.g., provided by the user device as described herein). For example, the receiving may be based at least partly on an association of the medical service and the second part of the medical platform. In one example, the clinician device may only then receive the medical service request when the medical service requested matches the second part of the medical platform.

The method may, for example, further comprise providing, by the clinician device, a response based at least partly on the medical service request.

Thereby, a direct, efficient, and time- and resource-saving communication between the clinician device and the user device is enabled.

In some examples, the method may further comprise providing, by the clinician device, additional medical patient data to the medical platform. For example, the providing of the additional medical patient data may be based at least partly on the second part.

Thereby, the overall versatility of the system may be increased as new data are allowed to enter the system from either end of the workflow. This may eventually improve the efficiency and accuracy of medical services as they may be based on a more complete database.

The additional medical patent data may, e.g., match the medical field associated with the second part. E.g., a clinician, being a radiologist or a dentist, may, e.g., be enabled to upload X-ray images while a clinician being an ophthalmologist may not be enabled to do so.

In some examples, the medical service request may comprise medical patient data and/or a request for analysis of said medical patient data. For example, the medical patient data may be anonymized based at least partly on the medical service request.

To ensure data protection, the clinician thus only receives anonymized data that only contains the information that is absolutely necessary for the medical service (comprising e.g., a diagnosis). E.g., in a first scenario, the age of the patient might be relevant for the requested service but the gender might not be. In this case, the anonymization may comprise removing any information on the patient's gender from the medical patient data provided with the request for the medical service. Thereby, a high degree of anonymization and data safety may be achieved without any negative effect on the requested medical service.

The medical patient data may be provided by a patient as a user, a clinician as a user, the medical platform database, and/or as the additional medical patient data which may join the medical patient data on the medical platform.

For example, the patient may be described in the medical service request as a `dummy patient' ("We report on the patient, "Dummy X, male, 50-60 years old, ..."). This means that the medical platform may convert the medical patient data into 'sample patients' which may be stored depending on the medical service request and may, e.g., comprise only the information on the patient that are relevant in the context of the associated medical service request. The anonymization may be dependent on the medical service request (e.g., age information: yes/no/only decade,...; with/without gender; with no previous history; ...). In the course of the anonymization, the (to be anonymized) medical patient data may accordingly be rewritten such that a "Dummy X" (= anonymized patient for anonymized medical patient data) in reality corresponds to patient "family name, first name, born on ..., ...". In the course of the anonymization, a medical patient data identifier may, e.g., be replaced with 'pseudo-identifier'. The medical platform may, e.g., comprise a module (e.g., a module for diagnostic support) which may store the assignment to the original identifier.

Further, the medial platform may comprise a database with predetermined (types of) medical service requests. Based on this information, the forwarded medical patient data may be reduced/redacted/generalized.

Each anonymized medical patient data (set) and/or medical service request may be assigned a unique identifier. E.g., upon receiving the response as described herein, the findings of the requested medical service may be reassigned to the real patient using the identifier and rewritten accordingly ("Dummy X" to "family name, first name, born on ..., ...".").

According to a third aspect, a method relating to the operation of the medical platform is described consistently with the involvement of the medical platform in the method according to the first and second aspects. Said third aspect relates to a method for central medical service mediation comprises: receiving, by a medical platform, a medical service request from a user device; receiving, by the medical platform, a user legitimation key; transmitting, by the medical platform, the medical service request securely to a clinician device; wherein the user legitimation key is configured to allow for a secure transmission of the medical service request by the medical platform.

According to a fourth aspect, a method for central medical service mediation comprises: receiving, by a medical platform, a clinician registration request from a clinician device; providing, by the medical platform, an instruction for a clinician qualification check; and granting, by the medical platform, clinician access, via the clinician device, to at least a second part of the medical platform upon successful execution of the instruction for the clinician qualification check.

The methods according to the third and fourth aspect achieve essentially the advantageous technical effects described herein in reference to the first and second aspects.

The method may for example further comprise receiving, by the medical platform, a response to a medical service request from a clinician device; and transmitting the response to a user device associated with the medical service request.

Thereby, the medical platform may act as a central node that may distribute and/or transmit the medical service requests and corresponding responses in a simple, yet efficient way. This may significantly improve the overall efficiency of the workflows around medical services.

A fifth aspect refers to a computer program comprising instruction for executing the steps of any of the methods described herein.

A computer program can be written in any form of programming language, including compiled or interpreted languages, and it can be deployed in any form, including as a stand-alone program or as a module, component, subroutine, or other unit suitable for use in a computing environment.

A sixth aspect of the present invention relates to a system for central medical service mediation comprising: at least one first part configured to receive a medical service request and a user legitimation key from a user device; at least one second part configured to receive a response to the medical service request from a clinician device; at least one transmission module configured to transmit the medical service request to the clinician device and to transmit the response to the medical service request to the user device; and at least one legitimation module configured to verify whether the user legitimation key is configured to allow for a secure transmission of the medical service request by the medical platform and to control the transmission of the medical service request based at least partly on the verification.

The system may, e.g., be or comprise the medical platform described herein.

The medical platform may, e.g., be hosted by and/or comprise one or more (e.g., cloud-based) servers/computers. For example, the one or more computers can be configured to be suitable for the execution of a computer program and can include, by way of example, both general and special purpose microprocessors, and any one or more processors of any kind of digital computer. Generally, a processor will receive instructions and data from a read-only storage area or a random access storage area or both. Elements of a computer system include one or more processors for executing instructions and one or more storage area devices for storing instructions and data. Generally, a computer system will also include, or be operatively coupled to receive data from, or transfer data to, or both, one or more machine-readable storage media, such as hard drives, magnetic disks, solid state drives, magneto-optical disks, or optical disks. Machine-readable storage media suitable for embodying computer program instructions and data include various forms of non-volatile storage area, including by way of example, semiconductor storage devices, e.g., EPROM, EEPROM, flash storage devices, and solid state drives; magnetic disks, e.g., internal hard disks or removable disks; magneto-optical disks; and CD-ROM, DVD-ROM, and/or Blu-ray discs.

In some implementations, the processes described above can be implemented using software for execution on one or more mobile computing devices, one or more local computing devices, and/or one or more remote computing devices (which can be, e.g., cloud computing devices). For instance, the software forms procedures in one or more computer programs that execute on one or more programmed or programmable computer systems, either in the mobile computing devices, local computing devices, or remote computing systems (which may be of various architectures such as distributed, client/server, grid, or cloud), each including at least one processor, at least one data storage system (including volatile and non-volatile memory and/or storage elements), at least one wired or wireless input device or port, and at least one wired or wireless output device or port.

In some implementations, the software may be provided on a medium, such as CD-ROM, DVD-ROM, Blu-ray disc, a solid state drive, or a hard drive, readable by a general or special purpose programmable computer or delivered (encoded in a propagated signal) over a network to the computer where it is executed. The functions can be performed on a special purpose computer, or using special-purpose hardware, such as coprocessors. The software can be implemented in a distributed manner in which different parts of the computation specified by the software are performed by different computers. Each such computer program is preferably stored on or downloaded to a storage media or device (e.g., solid state memory or media, or magnetic or optical media) readable by a general or special purpose programmable computer, for configuring and operating the computer when the storage media or device is read by the computer system to perform the procedures described herein. The inventive system can also be considered to be implemented as a computer-readable storage medium, configured with a computer program, where the storage medium so configured causes a computer system to operate in a specific and predefined manner to perform the functions described herein.

The medical platform and/or the system may, e.g., further comprise at least one of the following: a billing module configured to perform functions related to billing the medical services described herein, a client application for the diagnostic service providers, which signals the availability status of the diagnostic service provider to the cloud or server system (1) at any time and thus the diagnostic service can be provided in real time, a quality management module with feedback function on the quality of the individually provided medical service, a deletion module configured to delete medical patient data after completed medical service or expiry of a maximum storage period, an activity module configured to provide an activity logging (audit trail), a statistics and/or machine learning module that can provide support for the findings based on the patient data and findings/medical recommendations, an interface(s) to cost bearers, a module for controlling push media (SMS, WhatsApp, e-mail, ...), a translation module for translating the medical service request into the language of the clinician and/or of the response into the language of the user, and/or a status indication module providing a status of the service provider indicating until when a response can be provided by the service provider/clinician.

Generally, any means of the system described herein may be implemented as a step of a method and/or an instruction of a computer program and vice versa. As the different aspects described herein stand in a functional context with one another, any feature described herein in reference to either of the aspects may be isolated and/or implemented as a feature of any of the other aspects. The methods of the different aspects may be executed together and/or may be combined and/or at least (selected) steps thereof may be combined as to form one combined method.

The description refers, inter alia, to users and clinicians. It is not excluded that a clinician can use the described workflow/medical platform as a "user" and vice versa.

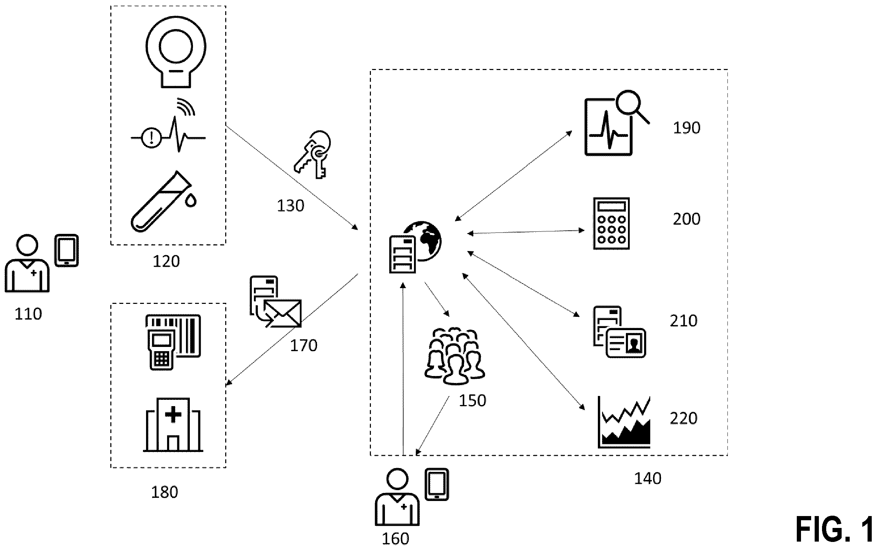

- Fig. 1: shows an exemplary system comprising the medical platform, the user device, and the clinician device.

Figure 1 shows the system according to the invention. An exemplary user device 110 provides a medical service request (e.g., comprising medical patient data, e.g., comprising a data set with diagnostic information) 120 via a secure and authenticated connection 130 to the medical platform 140. The medical service request may comprise a detailed request to have said medical patient data and/or the diagnostic information assessed by a qualified clinician and/or to make treatment recommendations.

The medical platform 140 may select an available clinician from a database of registered clinicians 150 or makes the request available to suitable clinicians 150, who can assign the request to themselves. The medical patient data request is then transmitted to a clinician device 160 of the selected qualified clinician, e.g., via download once said clinician checks their medical platform inbox (which may, e.g., be forwarded automatically as e-mail to the e-mail inbox of the clinician).

In the exemplary workflow of Fig. 1, the clinician analyses the diagnostic data and sends documentation of the findings and treatment as a response 180 to the requesting clinical user 110 via a secure interface 170. After completion of the diagnosis and data transfer to the first clinical user device 110, the medical patient data may automatically be deleted or anonymized in the medical platform 140 and/or throughout the system.

In addition, the medical platform 140 may comprise, as shown exemplarily in Fig. 1, one or more of the following modules: a module for diagnostic support 190, a billing module 200 for payment of the medical service, a registration portal 210 for registering the system users (users, patients, and clinicians) together with documentation of their medical qualifications and a machine learning module 220, which may derive rules for subsequent diagnostic support and quality assurance from the enquiries and findings. The medical platform 140 and/or the whole system may be implemented such that it may be certified in accordance with ISO 27001.

## Claims

1. Method for central medical service mediation, the method comprising:
providing, by a user device, a medical service request to a medical platform;
providing a user legitimation key to the medical platform;
wherein the user legitimation key is associated with the medical service request and/or the user device; and
wherein the user legitimation key is configured to allow for a secure transmission of the medical service request by the medical platform; and
receiving, by the user device, a response based at least partly on the medical service request.

2. The method of claim 1, wherein the user legitimation key comprises a time window defining the time during which the medical service request can be provided to the medical platform by the user device to allow for transmission of the medical service request by the medical platform.

3. The method of claim 2, further comprising authenticating, by the user device, of a user; and wherein the providing of the medical service request is based at least partly on the authenticating.

4. The method of claim 3, wherein the authenticating comprises:
providing, by the user device, an authentication request to an authentication module of the medical platform; and
being granted user access, via the user device, to at least a first part of the medical platform.

5. Method for central medical service mediation, the method comprising:
providing, by a clinician device, a clinician registration request to a medical platform;
receiving, by the clinician device, an instruction for a clinician qualification check; and
being granted clinician access, via the clinician device, to at least a second part of the medical platform upon successful execution of the instruction for the clinician qualification check.

6. The method of claim 5, wherein the second part of the medical platform is based at least partly on the clinician registration request, the instruction and/or the successful execution.

7. The method of claim 5 or 6, further comprising receiving, by the clinician device, a medical service request from the medical platform; wherein the receiving is based at least partly on an association of the medical service and the second part of the medical platform.

8. The method of claim 7, further comprising providing, by the clinician device, a response based at least partly on the medical service request.

9. The method of any of claims 5 to 8, further comprising providing, by the clinician device, additional medical patient data to the medical platform; wherein the providing of the additional medical patient data is based at least partly on the second part.

10. The method of any of claims 1 to 4 and 7 to 9, wherein the medical service request comprises medical patient data and/or a request for analysis of said medical patient data; and wherein preferably the medical patient data are anonymized based at least partly on the medical service request.

11. Method for central medical service mediation, the method comprising:
receiving, by a medical platform, a medical service request from a user device;
receiving, by the medical platform, a user legitimation key;
transmitting, by the medical platform, the medical service request securely to a clinician device;
wherein the user legitimation key is configured to allow for a secure transmission of the medical service request by the medical platform.

12. Method for central medical service mediation, the method comprising:
receiving, by a medical platform, a clinician registration request from a clinician device;
providing, by the medical platform, an instruction for a clinician qualification check; and
granting, by the medical platform, clinician access, via the clinician device, to at least a second part of the medical platform upon successful execution of the instruction for the clinician qualification check.

13. The method of any of claims 11 to 13, further comprising:
receiving, by the medical platform, a response to a medical service request from the clinician device; and
transmitting the response to the user device associated with the medical service request.

14. Computer program comprising instructions for executing the steps of the method of any of claims 1 to 13.

15. System for central medical service mediation, the system comprising:
at least one first part configured to receive a medical service request and a user legitimation key from a user device;
at least one second part configured to receive a response to the medical service request from a clinician device;
at least one transmission module configured to transmit the medical service request to the clinician device and to transmit the response to the medical service request to the user device; and
at least one legitimation module configured to verify whether the user legitimation key is configured to allow for a secure transmission of the medical service request by the medical platform and to control the transmission of the medical service request based at least partly on the verification.
